(19) Europäisches Patentamt
European Patent Office
Office européen des brevets

(11) Publication number: **0 044 090**
**A2**

# (12) EUROPEAN PATENT APPLICATION

(21) Application number: 81107055.6

(22) Date of filing: 28.09.79

(51) Int. Cl.[3]: C 07 C 103/52
A 61 K 31/13, C 07 D 401/14
C 07 D 501/22, C 07 J 43/00

(30) Priority: 02.10.78 US 947374

(43) Date of publication of application: 20.01.82 Bulletin 82/3

(84) Designated Contracting States: AT BE CH DE FR GB IT LU NL SE

(60) Publication number of the earlier application in accordance with Art. 76 EPC: 0 009 944

(71) Applicant: MERCK & CO. INC.
126, East Lincoln Avenue P.O. Box 2000
Rahway, New Jersey 07065(US)

(72) Inventor: Firestone, Raymond A.
60 Hunter Avenue
Fanwood New Jersey 07023(US)

(74) Representative: Crampton, Keith John Allen et al,
D. Young & Co. 10 Staple Inn
London WC1V 7RD(GB)

## (54) Lysosomotropic detergent therapeutic agents, compositions containing them and their uses.

(57) Compounds of formula:

M–N=A

in which M is a peptide substituent comprising z-gly-phe, gly-phe, z-arg-arg, or benzoyl-arg, and A or N=A is an active lysosome-inhibiting substance comprising a known therapeutic drug residue or an amino radical of the formula:

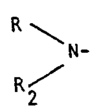

in which R is an alkyl or substituted alkyl substituent of from 8 to 18 carbons and $R_2$ is fluoroalkyl or hydrogen, are useful in the control of fertility, in the inhibition of malignant cell growth, and in the control of other diseases which accumulate macrophages at the disease site. They can be incorporated in pharmaceutical compositions for this purpose.

EP 0 044 090 A2

# LYSOSOMOTROPIC DETERGENT THERAPEUTIC AGENTS, COMPOSITIONS CONTAINING THEM AND THEIR USES

This invention relates to compositions useful in the control of fertility, in the inhibition of malignant cell growth, and in the control of other diseases which accumulate macrophages at the disease site.

Lysosomotropic substances have been designated as substances which are selectively taken up by lysosomes regardless of their chemical nature or mechanism of uptake. It has been suggested that the property of lysosomotropism is useful in the preparation of therapeutic agents having useful biological properties. These substances can enter the lysosomes preferentially and exert their biological influence because of their own unique biological activity. They also can be coupled with active drugs and, following preferential uptake by lysosomes, can be hydrolysed within the lysosome with release of the active drug at the target area. Thus, in one method of using the lysosomotropic agent DNA, a complex of DNA with the potent cytotoxic agent daunorubicin was formed. It was then found that the complex was preferentially taken up by the tumour cells and that the DNA was preferentially degraded by lysosomes leaving the active daunorubicin within the lysosome. A drawback of this particular method is that the toxic dose is very close to the therapeutic dose which makes the margin of safety very narrow.

The present invention is concerned with the inhibition of lysosome-bearing cells and involves the use of new compositions and

new pharmaceutical formulations useful in inhibiting the function of lysosome-bearing cells.

Still more specifically, the present invention provides a compound of the formula :

$$M-N=A$$

in which M is a peptide substituent comprising z-gly-phe, gly-phe, z-arg-arg, or benzoyl-arg, and A or N=A is an active lysosome-inhibiting substance comprising a known therapeutic drug residue or an amino radical of the formula :

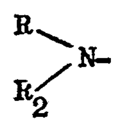

in which R is an alkyl or substituted alkyl substituent of from 8 to 18 carbons and $R_2$ is fluoroalkyl or hydrogen and is usually coupled to M by means of an amide linkage.

Compounds of the type :

$$M-N=A$$

are readily prepared by reaction of the selected amine with a selected peptide in the presence of a substance such as dicyclocarbodiimide under anhydrous conditions.

The present invention also provides a pharmaceutical composition comprising a said compound of formula :

$$M-N=A$$

and a pharmaceutical carrier.

Compounds of the present invention are illustrated by the following structural formulae in which the M substituent is a peptide as defined above :

Peptide derivatives of antibiotic 593A and analogs as disclosed in U.S. Patents US-A-3,718,651 and US-A13,840,542:

BAD ORIGINAL

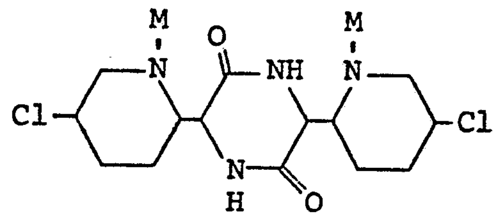

;

and peptide derivatives of a cephalosporin compound of the formula :

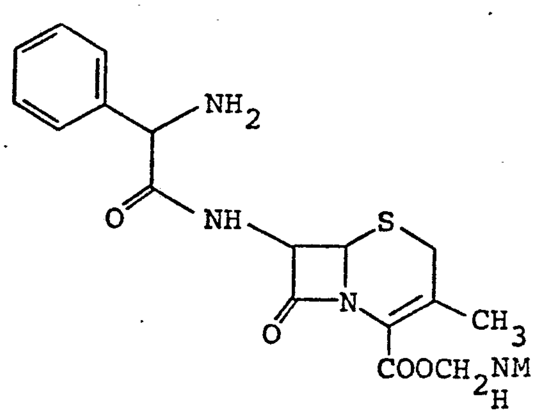

or an antiinflammatory steroid of the formula :

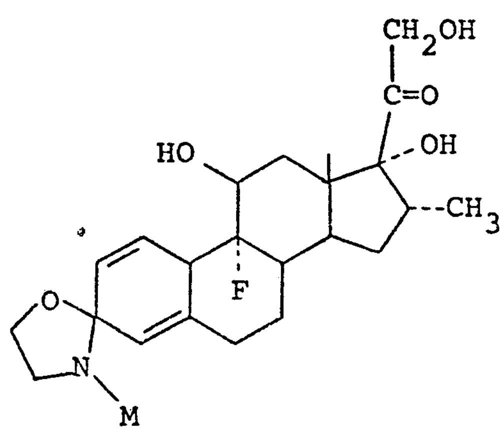

The compounds of the present invention can readily be hydrolysed by lysosomal enzymes either within the lysosome or at the outside surface of the lysosome-bearing cell.

The A or -N=A substituent is either an antifertility agent, a tumour-inhibiting substance, an antibiotic or an antiinflammatory steroid.

The compounds of the present invention are useful in selectively killing or altering the function of lysosome-bearing cells, and they are also useful as systems for the delivery of an active drug to the disease site where the active drug is released by lysosomal enzymatic hydrolysis. Thus, some compounds of this invention are useful as antifertility agents. As antifertility agents, the active principle is incorporated into vaginal or other contraceptive creams, jellies or foams, in a concentration of from 0.1% to 10%, preferably 1-10%. The formulation prepared in this manner is administered intravaginally and contact of the sperm with the composition of the present invention incorporates the active principle into the acrosome of the sperm cells, thus rendering them infertile.

Certain compounds of the present invention are also useful in inhibiting the growth of malignant tumour cells. For this purpose, the compounds of the present invention are administered either orally or by injection in a dosage schedule of 25-500 mg/kg/day.

Certain compounds of the present invention are also useful in combating infection caused by resistant microorganisms, which are sequestered within lysosomes and thus unavailable to attack by antibiotics. The lysosomotropic compounds of the present invention comprise an antibiotic incorporating a weakly basic amine group and a peptide linked to a carboxylic function of the antibiotic through an amide function. Such compounds are incorporated into the cell lysosome where they are hydrolysed by lysosomal enzymes, thus releasing the active antibiotic principle to attack the infecting microorganism.

Certain of the compounds of the present invention are useful in the treatment of inflammation. Thus a composition of an antiinflammatory steroid coupled to a lysosomally cleavable peptide through an amine function of the steroid is attracted to the site of

BAD ORIGINAL

inflammation, where it is cleaved by lysosomal enzymes at the site of inflammation thereby producing the desired antiinflammatory effect with reduced side effects.

The compounds of this invention can be administered either topically or systemically, i.e., intravenously, subcutaneously, intramuscularly, orally, rectally, by aerosolization, or in the form of sterile implants for long action.

The pharmaceutical compositions can be sterile, injectable suspensions or solutions, or solid, orally administrable, pharmaceutically acceptable tablets or capsules; the compositions can also be intended for sublingual administration, or for suppository use. It is especially advantageous to formulate compositions in dosage unit forms for ease and economy of administration and uniformity of dosage. "Dosage unit form" as a term used herein refers to physically discrete units suitable as unitary dosages for animal and human subjects, each unit containing a predetermined quantity of active material calculated to produce the desired biological effect in association with required pharmaceutical means.

The low cost and ready accessibility of the compositions of this invention make them particularly promising for applications in veterinary medicine in which field their utilities are comparable to those in human medicine.

## CLAIMS

1. A compound of formula :

M-N=A

in which M is a peptide substituent comprising z-gly-phe, gly-phe, z-arg-arg, or benzoyl-arg, and A or N=A is an active lysosome-inhibiting substance comprising a known therapeutic drug residue or an amino radical of the formula :

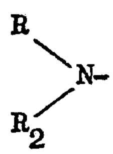

in which R is an alkyl or substituted alkyl substituent of from 8 to 18 carbons and $R_2$ is fluoroalkyl or hydrogen.

2. A pharmaceutical composition comprising a compound as claimed in Claim 1 together with a pharmaceutical carrier.

3. A method for inhibiting or altering the function of lysosome-bearing cells that comprises contacting the said cells with a pharmaceutical composition comprising a pharmaceutical carrier and a lysosomotropic substance comprising a peptide conjugate hydrolysable by lysosomal enzymes to a biologically active substance.

BAD ORIGINAL

4. For use in inhibiting or altering the function of lysosome-bearing cells, a compound as claimed in Claim 1 or a composition as claimed in Claim 2.